# NOUVEAU FASCICULE DE BREVET EUROPEEN

(11) **EP 0 987 236 B2**
(45) Date de publication et mention de la décision concernant l'opposition: **20.01.2016**
(45) Mention de la délivrance du brevet: 02.05.2003
(21) Numéro de dépôt: 99402245.7
(22) Date de dépôt: 13.09.1999
(51) Int. Cl.: C07C 1/04, C10G 2/00

(54) **Procédé de conversion du gaz de synthèse en présence d'un catalyseur à base de métal du groupe VIII, les particules du métal étant réparties sour forme d'agrégats**
Verfahren zur Umsetzung von Synthesegas in Gegenwart eines Katalysators der ein Metall der Gruppe VIII enthält indem die Metallteilchen als Aggregate verbreitet sind
Process for the conversion of synthesis gas in presence of a catalyst comprising a metal of group VIII, its particles being spread as aggregates

(30) Priorité: 18.09.1998 FR 9811723
(43) Date de publication de la demande: 22.03.2000
(73) Titulaire: IFP Énergies nouvelles, 92852 Rueil-Malmaison Cedex (FR); ENI S.p.A., Roma (IT)
(72) Inventeur: Roy, Magalie, 92500 Rueil Malmaison (FR); Marion, Marie-Claire, 69390 Vernaison (FR)
(74) Mandataire: Cabinet Plasseraud

(56) Documents cités:
- EP-A- 0 174 696
- EP-A1- 0 174 696
- US-A- 4 801 573
- Statoils GMD, Gas to middle distillates, process, P Roterud et al., Presentationat SPUNG gas utilisation seminar, Trondheim, September 1989
- CHUNG J.S.: 'Preparation and Fischer-Tropsch reaction of highly-reduced cobalt clusters in cobalt-exchanged zeolite' JOURNAL OF MOLECULAR CATALYSIS vol. 79, 1993, pages 199 - 215

## Description

La présente invention concerne l'utilisation d'un catalyseur comprenant au moins un support, au moins un métal du groupe VIII sous forme de particules métalliques, dans un procédé de synthèse d'hydrocarbures à partir d'un mélange comprenant CO-(CO₂)-H₂ (c'est à dire un mélange comprenant CO-H₂ comprenant éventuellement du CO₂, appelé gaz de synthèse), plus particulièrement l'utilisation permettant de réaliser la conversion du gaz de synthèse en mélange d'hydrocarbures linéaires saturés essentiellement constitués d'hydrocarbures C5⁺ (c'est-à-dire possédant au moins 5 atomes de carbone par molécule), ou plus précisément en un mélange d'hydrocarbures, essentiellement linéaires et saturés, contenant au moins 25% poids d'hydrocarbures C5⁺ par rapport à l'ensemble des hydrocarbures formés. L'invention concerne également le catalyseur utilisé ainsi que son procédé de préparation.

Il est connu de l'homme du métier que le gaz de synthèse peut être converti en hydrocarbures en présence de catalyseur contenant des métaux de transition. Cette conversion opérée à haute température et sous pression est connue dans la littérature sous le nom de synthèse Fischer-Tropsch. Ainsi des métaux du groupe VIII de la classification périodique des éléments tels que le fer, le ruthénium, le cobalt et le nickel catalysent la transformation de mélanges CO-(CO₂)-H₂ (c'est-à-dire un mélange CO-H₂ comprenant éventuellement du CO₂, appelé gaz de synthèse) en hydrocarbures liquides et/ou gazeux.

Les produits préparés par synthèse Fischer Tropsch en présence de catalyseurs comprenant des métaux du groupe VIII présentent une distribution très large en terme de poids moléculaire. Ainsi seule une faible proportion de produits obtenus se situent dans la gamme des distillats moyens constitués par des fractions kérosène et gasoil, la (ou les) fraction(s) kéronsène étant constituée(s) par un mélange d'hydrocarbures dont les points d'ébullition sont compris entre 140°C et 300 °C, et la(ou les) fraction(s) gasoil étant constituée(s) par un mélange d'hydrocarbures de points d'ébullition compris entre 180°C et 370°C lors d'une distillation atmosphérique telle que réalisée par l'homme du métier sur un brut pétrolier.

Des efforts importants ont été entrepris depuis 1973 afin d'améliorer le rendement des distillats moyens basés sur la conversion du gaz de synthèse.

Différentes méthodes ont été décrites et développées dans l'art antérieur destinées à améliorer la répartition du métal de manière à limiter les phénomènes diffusionnels à l'intérieur du grain. Le support est alors enduit ou enrobé de manière à ce que le métal reste à la périphérie du grain.

La demande de brevet EP 0174696 A décrit un procédé de préparation d'un catalyseur Fischer-Tropsch à base de cobalt, où le cobalt est distribué de telle façon que ΣVp/Σ Vc < 0,85 (ΣVp étant la quantité de Co contenu dans la périphérie du solide, ΣVc étant la quantité de Co contenu dans la totalité du solide). L'inventeur montre que cette distribution favorise la formation de C₅₊. Le catalyseur est préparé par imprégnation du support (préférentiellement la silice) déjà immergé dans l'eau pendant 30 secondes et peut contenir un promoteur de préférence le zirconium.

Le brevet US 5,036,032 décrit la préparation d'un catalyseur à base de cobalt permettant d'augmenter la dispersion du cobalt sur la surface externe du support. La méthode de préparation permet d'augmenter la densité de sites actifs en surface en utilisant un précurseur de viscosité suffisante pour empêcher la pénétration dans les pores par capillarité. La réduction directe sans étape de calcination permet d'augmenter la dispersion. SiO₂ est préféré comme support. Les limitations diffusionnelles sont ainsi évitées.

Le brevet US 4,977,126 décrit également une méthode de dispersion en surface par vaporisation d'un liquide dans lequel le composé métallique est dissout. Une couche périphérique est ainsi formée.

D'autres brevets US 4,605,679, US 4,729,981, EP 0 535 790 A décrivent des modifications de l'étape de réduction / activation.

Enfin la demande de brevet EP 0 736 326 A décrit une nouvelle méthode de préparation du catalyseur au cobalt comprenant une étape d'imprégnation suivie d'une étape de séchage du catalyseur sous une pression inférieure à la pression atmosphérique.

L'influence de la taille des particules sur l'activité du catalyseur a largement été débattue dans la littérature. Dans le cas de la transformation du gaz de synthèse en hydrocarbures liquides et/ou gazeux, il a été montré que l'activité spécifique était indépendante de la dispersion du métal et de la nature du support [E. Iglesia, Applied Catalysis A : General 161 (1997) 59].

L'activité spécifique correspond à l'activité du catalyseur ramené au nombre d'atomes métalliques accessibles au nombre de molécules à transformer. Le nombre d'atomes métalliques accessibles peut-être déterminé par des techniques de chimisorption de molécules sondes (oxygène, hydrogène, monoxyde de carbone) ou à partir de la taille des particules déterminée par microscopie électronique Ces différentes techniques sont bien connues de l'homme du métier.

La présente invention concerne un procédé de synthèse d'hydrocarbures à partir d'un mélange comprenant du monoxyde de carbone et de l'hydrogène CO-H₂, éventuellement du dioxyde de carbone CO₂, en présence d'un catalyseur comprenant un support et des particules d'au moins un métal du groupe VIII de la classification périodique des éléments déposé(s) sur le support, caractérisé en ce qu'au moins une partie des particules du métal du groupe VIII du catalyseur ne sont pas isolées les unes par rapport aux autres.

La demanderesse a découvert de manière surprenante que lorsque au moins une partie des particules métalliques n'est pas isolée par rapport aux autres particules déposées sur le support mais que les particules forment des agglomérats ou agrégats, le catalyseur présente une sélectivité en C₅₊ améliorée par rapport à un catalyseur qui présente des particules isolées. Les particules du catalyseur selon l'invention ont généralement une taille comprise entre 50 et 500 Å, de préférence entre 60 et 400 Å. Le catalyseur selon l'invention présente une sélectivité en C₅₊ améliorés par rapport à un catalyseur comprenant des particules isolées de taille comprise entre 50 et 500 Å et par rapport à un catalyseur présentant des particules isolées de taille supérieures à 500 Å. Ces particules peuvent être réparties à la périphérie ou à l'intérieur des pores du support ou à la fois à la périphérie et à l'intérieur des pores.

L'état d'agrégation entre des particules de métal est défini par l'existence d'au moins une zone de contact entre au moins deux particules. L'état d'agrégation est caractérisé par une analyse par microscopie à transmission du catalyseur avant ou après réduction de la phase active.

Pour le catalyseur selon l'invention, l'analyse par microscopie montre qu'au moins 60% des particules, de préférence 80% des particules et de manière encore plus préférée 90% des particules présentent au moins une zone de contact avec au moins une autre particule, et de préférence avec au moins deux autres particules. Les particules sont préférentiellement déposées sous forme d'agglomérats sensiblement sphériques de taille comprise entre 500 et 100 000 Å. Elles peuvent également être disposées sous forme de grappes ou de chapelets ou tout autre configuration de manière à avoir au moins une zone de contact avec au moins une autre particule de préférence avec deux autres particules.

La présente invention présente un catalyseur utilisé en synthèse Fischer Tropsch dont les performances sont particulièrement stables et qui conduit après réduction sous hydrogène à la conversion du gaz de synthèse en un mélange d'hydrocarbures linéaires et saturés contenant au moins 50% poids d'hydrocarbures C5⁺ et moins de 20% de méthane par rapport à l'ensemble des hydrocarbures formés.

Les conditions de mise en oeuvre desdits catalyseurs pour la synthèse d'hydrocarbures sont habituellement les suivantes :

Le catalyseur comprenant au moins un métal du groupe VIII imprégné sur un support est séché puis calciné. Ensuite le catalyseur est pré-réduit par au moins un composé reducteur, par exemple choisi dans le groupe formé par l'hydrogène, le monoxyde de carbone et l'acide formique, éventuellement mis en contact avec un gaz inerte (azote par exemple dans un rapport molaire composé réducteur/(composé réducteur + gaz inerte) compris entre 0,001 :1 à 1 :1.

La réduction est menée en phase aqueuse entre 100°C et 600°C, de préférence entre 150°C et 400°C, entre 0,1 et 10 MPa et à une vitesse volumétrique horaire entre 100 et 40 000 volumes de mélange par volume de catalyseur et par heure. Cette réduction peut également être menée en phase liquide, le catalyseur étant mis en suspension dans un solvant inerte, par exemple une coupe paraffinique comprenant au moins un hydrocarbure ayant au moins 5, de préférence au moins 10 atomes de carbone par molécule si par la suite la réaction de synthèse d'hydrocarbures se déroule en phase liquide comprenant au moins un hydrocarbure ayant au moins 5, de préférence au moins 10 atomes de carbone par molécule.

La conversion du gaz de synthèse en hydrocarbures est ensuite opérée sous une pression totale habituellement comprise entre 0,1 et 15 MPa et de préférence entre 1 et 10 MPa, la température étant généralement comprise entre 150 et 350°C et de préférence entre 170 et 300°C.

La vitesse volumétrique horaire est habituellement comprise entre 100 et 20000 volumes de gaz de synthèse par volume de catalyseur et par heure et de préférence entre 400 et 5000 volume de gaz de synthèse par volume de catalyseur et par heure, et le rapport H₂/CO dans le gaz de synthèse est habituellement compris entre 1 : 2 et 5 :1, de préférence entre 1.2 :1 et 2.5 :1.

Le catalyseur est généralement utilisé en poudre fine calibrée (10-700 microns environ) ou en particules de diamètre équivalent compris entre 2 et 10 mm environ, respectivement en présence d'une phase liquide (dans les conditions opératoires), ou d'une phase gazeuse. La phase liquide peut être constituée par au moins un hydrocarbures ayant au moins, de préférence au moins 10, atomes de carbones par molécules.

L'élément du groupe VIII de la classification périodique des éléments est choisi parmi le fer, le cobalt et le ruthénium. De préférence, le métal du groupe VIII est le cobalt.

Le support du catalyseur selon l'invention comporte au moins un oxyde réfractaire généralement choisi parmi les oxydes de magnésium, d'aluminium, de silicium ou de zirconium pris seuls, en mélange entre eux ou avec des oxydes d'autres éléments de la classification périodique. Le support utilisé sera préférentiellement l'alumine. Le charbon, les alumino-silicates, les argiles ou tout autre composé pouvant servir comme support peut aussi être utilisé. Ce support peut être utilisé sous forme de poudre ou après mise en forme ; toute technique de mise en forme convient à l'invention.

L'introduction du métal du groupe VIII se fait de façon à obtenir généralement des particules de taille comprise entre 50 et 500 Å dispersées sur un support de telle façon qu'au moins 80% des particules et de manière encore plus préférée au moins 90% des particules présentent au moins une zone de contact avec au moins une autre particules et de préférence avec au moins deux autres particules.

Une technique de préparation du catalyseur qui convient particulièrement est l'imprégnation d'une solution contenant des particules d'oxyde de métal et/ou des particules de métal à déposer en suspension. Le solvant pourra être un solvant aqueux, par exemple l'eau, ou un solvant organique.

Afin d'augmenter les zones de contact entre les particules, la solution peut contenir une concentration limitée en agent complexant permettant de la stabiliser, l'agent complexant n'étant toutefois pas indispensable.

Une autre technique de préparation du catalyseur consiste à imprégner à l'aide d'une solution aqueuse d'un précurseur du métal du groupe VIII de la classification périodique des éléments, par exemple une solution aqueuse de sels tels que le nitrate de cobalt et l'acétate de cobalt.

L'imprégnation peut se faire goutte à goutte sur le support lui même chauffé à une température comprise entre la température ambiante et 200°C, induisant ainsi I 'évaporation de la solution aqueuse dès le contact avec le support.

Après dépôt par imprégnation des solutions, le catalyseur est généralement séché sous courant d'air ou d'azote à une température comprise entre 80°C et 120°C, éventuellement calciné sous courant d'air ou d'azote à des températures comprises entre 120°C et 500°C et réduit à des températures comprises entre 100°C et 500°C.

La teneur en poids par rapport au poids total de catalyseur en métal du groupe VIII est généralement comprise entre 0,1 et 50%, préférentiellement entre 1 et 30%.

Le catalyseur peut aussi contenir d'autres éléments additionnels tels que par exemple au moins un métal alcalin, des promoteurs tels que par exemple au moins un élément choisi parmi le ruthénium, le molybdène et le tantale. La teneur en poids d'un élément additionnel par rapport au poids total de catalyseur est généralement comprise entre 0,01 et 5%. Ces éléments additionnels peuvent être introduits en même temps que le métal du groupe VIII ou dans au moins une étape ultérieure.

Dans un mode particulier de réalisation de l'invention, le catalyseur contient du cobalt et du ruthénium.

Dans un autre mode particulier de réalisation de l'invention le catalyseur contient du cobalt et du tantale.

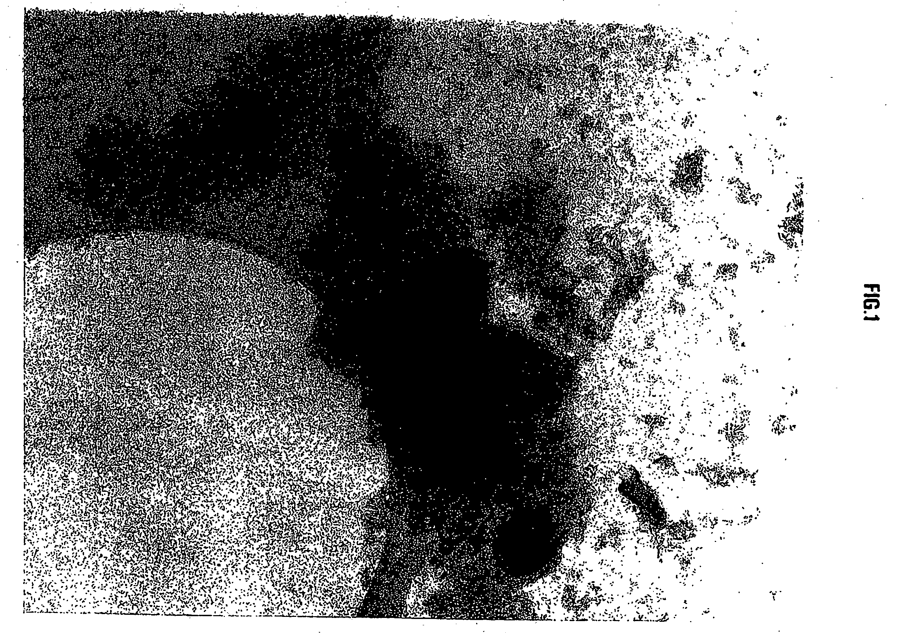

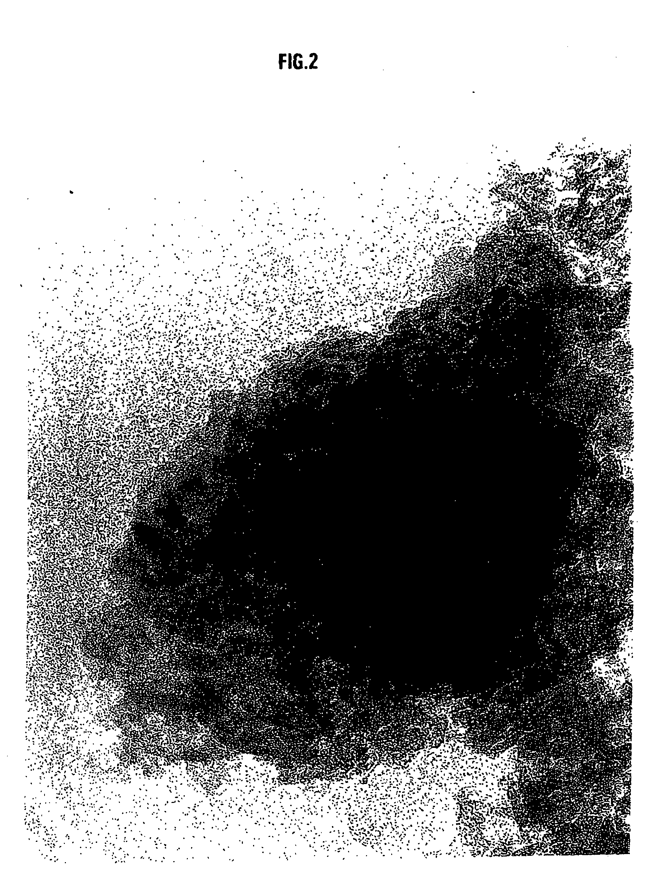

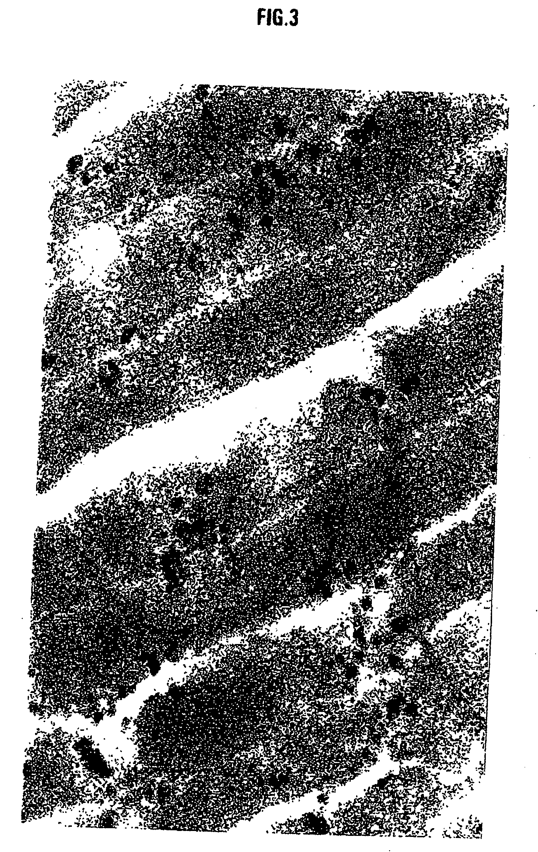

Pour illustrer l'invention, un ensemble de clichés représentatifs de catalyseurs selon l'invention sont donnés en annexe. Les figures 1 et 2 montrent des agrégats de particules de forme variées. La figure 3 est donnée à titre de comparaison et montre des particules isolées.

Les exemples qui suivent illustrent l'invention.

### Exemple 1 (selon l'invention) : catalyseur A

Un catalyseur A Co/Al2O3 est préparé par imprégnation d'une poudre d'alumine de surface spécifique de 180 m²/g. Ce support se présente sous forme de poudre de granulométrie comprise entre 10 et 150 microns.
Ce support est chauffé à 100°C sur un drageoir tournant et en même temps est imprégné par un solution aqueuse de nitrate de cobalt.
Après imprégnation, le support est calciné à 400°C.

La teneur finale en cobalt est de 12,5%.
Les particules de cobalt ont une taille comprise entre environ 150 et 180 Å.
L'analyse par microscopie à transmission de ce catalyseur montre que 98% des particules de cobalt forment des agrégats sphériques de tailles comprises entre 300 et 4000 Å.

### Exemple 2 (comparatif) : catalyseur B

Un catalyseur B est préparé à partir du support décrit dans l'exemple A.
Ce support est imprégné par un solution aqueuse de nitrate de cobalt à température ambiante. Après imprégnation, le support est séché à 120°C et calciné à 400°C.
La teneur finale en cobalt est de 13%.
L'analyse microscopique montre la présence de particules de cobalt de taille supérieure à 500 Å à 100% isolées et d'autres particules de tailles comprises entre 150 et 300 Å, isolées.

### Exemple 3 (comparatif) : catalyseur C

Un catalyseur C, Co/SiO2, est préparé par la méthode sol gel décrite dans le brevet US 5,302,622.
La surface spécifique est de 250 m2/g.
Le teneur en cobalt est de 25%.
Les particules de cobalt ont des tailles comprises entre 130 et 200 Å avec quelques particules de tailles supérieures à 580 Å.
45% des particules de cobalt se trouvent isolées.

### Exemple 4 (selon l'invention) : catalyseur D

Un catalyseur D est préparé à partir du support décrit dans l'exemple A.
Le cobalt est imprégné dans une première étape à partir d'une solution de nitrate de cobalt. On laisse ensuite le solide mûrir à température ambiante pendant 12 heures .
Le solide est ensuite séché à 120°C et calciné à 400°C.
Le ruthénium est ensuite imprégné en solution aqueuse.
Le solide est séché à 110°C et calciné à 300°C.
La teneur en cobalt est de 15% et la teneur en ruthénium est de 0.25%.
99% des particules de cobalt obtenues ont des tailles comprises entre entre 150 et 300 Å et forment des agglomérats.

### Exemple 5 (comparatif) : catalyseur E

Un catalyseur E est préparé à partir du support décrit dans l'exemple A.
Le cobalt est imprégné dans une première étape à partir d'une solution de nitrate de cobalt. Le solide est ensuite séché à 120°C et calciné à 400°C puis réduit dans un réacteur tubulaire sous hydrogène pur à 400°C et passivé sous oxygène.
Le ruthénium est ensuite imprégné en solution aqueuse. Le solide est séché à 110°C, calciné à 300°C et à nouveau réduit dans un réacteur tubulaire sous hydrogène pur à 400°C et passivé sous oxygène.
La teneur en cobalt est de 15% et la teneur en ruthénium est de 0.25%.
Les particules de cobalt obtenues ont des petites tailles comprises entre 50 et 100 Å et 42% des particules sont isolées.

### Exemple 6: Tests catalytiques

Les catalyseurs A, B, C, D et E dont les préparations sont décrites dans les exemples 1 à 5 ci-dessus sont testés en lit fixe phase gazeuse dans une unité fonctionnant en continu et opérant sur 20 cm³ de catalyseur.
Les catalyseurs sont préalablement réduits in situ à 350°C d'abord pendant 12 h sous un mélange d'hydrogène et d'azote contenant 30% d'hydrogène, puis pendant 12h sous hydrogène pur.
Les conditions de test des catalyseurs sont les suivantes :
- T°C = 220°C,
- Pression = 2MPa
- vitesse volumique horaire (VVH) = 1500 h⁻¹
- rapport molaire H₂/CO = 2/1

**TABLEAU :**

| **Conversion du gaz de synthèse en hydrocarbures** | | | | | | |
|---|---|---|---|---|---|---|
| **Catalyseur** | **% de particules isolées** | **composition** | **Conv CO (%vol après 100h)** | **Distribution des produits formés (% poids)** | | |
| | | | | **C1** | **C1-C4** | **C5+** |
| A (invention) | 2% | Co | 55 | **12,5** | **25,2** | **74,8** |
| B(comparatif) | - | Co | 70 | **21** | **35,5** | **64,5** |
| C(comparatif) | 45% | Co | 68 | **33** | **45** | **55** |
| D(invention) | 1% | Co-Ru | 65 | **11** | **22,5** | **77,5** |
| E(comparatif) | 42% | Co-Ru | 45 | **26,5** | **41,5** | **58,5** |

Les résulats du tableau montrent que le procédé selon l'invention en présence d'un catalyseur dont la majeure partie des particules métalliques ne sont pas isolées mais sous forme d'agrégats présente une séléctivité en C5+ améliorée par rapport aux catalyseurs de l'art antérieur.

## Revendications

1. Procédé de synthèse d'hydrocarbures à partir d'un mélange comprenant du monoxyde de carbone et de l'hydrogène CO-H2, en présence d'un catalyseur comprenant un support, des particules d'au moins un métal du groupe VIII de la classification périodique des éléments déposé(s) sur le support, **caractérisé en ce qu'**au moins 80% des particules métalliques du catalyseur présente au moins une zone de contact avec au moins une autre particule et **en ce que** lesdites particules ont une taille comprise entre 50 et 500 A.

2. Procédé selon la revendication 1, tel qu'au moins 90% des particules métalliques du catalyseur présente au moins une zone de contact avec au moins une autre particule.

3. Procédé selon l'une des revendications 1 ou 2, tel que le métal du groupe VIII du catalyseur est le cobalt.

4. Procédé selon l'une des revendications 1 à 3, tel que le catalyseur contient en outre du ruthénium.

5. Procédé selon l'une des revendications 1 à 4, tel que la pression totale est comprise entre 0,1 et 15 MPa, la température est comprise entre 150 et 350°C, avec une vitesse volumétrique horaire comprise entre 100 et 20 000 volumes de gaz de synthèse par volume de catalyseur et par heure, avec un rapport H2/C0 dans le gaz de synthèse compris entre 1 : 2 et 5 : 1, de préférence entre 1,2 : 1 et 2,5 : 1.

6. Procédé selon l'une des revendications 1 à 5, dans lequel les particules métalliques ont une taille comprise entre 60 et 400 A.

7. Procédé selon l'une des revendications 1 à 6 dans lequel les particules sont sous forme d'agglomérats sensiblement sphériques de taille comprise entre 500 et 100.000 A.

8. Préparation d'un catalyseur comprenant un support, des particules d'au moins un métal du groupe VIII de la classification périodique des éléments déposé(s) sur le support, et dans lequel au moins 80 % des particules métalliques du catalyseur présente au moins une zone de contact avec au moins une autre particule et lesdites particules ont une taille comprise entre 50 et 500 A, au moyen d'un procédé comprenant au moins une étape d'imprégnation au moyen d'une solution d'un précurseur dudit métal sur un support chauffé à une température comprise entre la température ambiante et 200°C de manière à induire l'évaporation de la solution dès le contact avec le support.

9. Procédé selon l'une des revendications 1 à 8 dans lequel l'imprégnation dudit catalyseur est réalisée goutte à goutte.

## Patentansprüche

1. Verfahren zur Synthese von Kohlenwasserstoffen aus einem Gemisch, das Kohlenmonoxyd und Wasserstoff CO-H₂ umfasst, in Gegenwart eines Katalysators, der einen Träger und Partikel wenigstens eines Metalls der Gruppe VIII des Periodensystems der Elemente, abgeschieden auf dem Träger umfasst, **dadurch gekennzeichnet, dass** wenigstens 80% der metallischen Katalysatorpartikel wenigstens eine Kontaktzone mit wenigstens einem anderen Partikel aufweist, und dadurch, dass die Partikel einen Umfang zwischen 50 und 500 Å haben.

2. Verfahren nach Anspruch 1, **dadurch gekennzeichnet, dass** wenigstens 90 % der metallischen Katalysatorpartikel wenigstens eine Kontaktzone mit wenigstens einem anderen Partikel aufweist.

3. Verfahren nach einem der Ansprüche 1 oder 2, derart, dass das GruppeVIII-Metall des Katalysators Kobalt ist.

4. Verfahren nach einem der Ansprüche 1 bis 3, derart, dass der Katalysator im uebrigen Ruthenium enthaelt.

5. Verfahren nach einem der Ansprüche 1 bis 4, derart, dass der Gesamtdruck zwischen 0,1 und 15 MPa liegt, die Temperatur zwischen 150 und 350°C mit einer stündlichen Volumengeschwindigkeit zwischen 100 und 20000 Synthesegasvolumen pro Katalysator-Volumen und pro Stunde bei einem Verhältnis H₂/CO in dem Synthesegas zwischen 1: 2 und 5: 1, vorzugsweise zwischen 1,2: 1 und 2,5: 1 liegt.

6. Verfahren nach einem der Ansprüche 1 bis 5, bei dem die metallischen Partikel einen Umfang zwischen 60 und 400 Å haben.

7. Verfahren nach einem der Ansprüche1 bis 6, bei dem die Partikel in Form von Agglomeraten sind, die im wesentlichen kugelförmig von einem Umfang zwischen 500 und 100 000 Å sind.

8. Herstellung eines Katalysators, der einen Träger, Partikel wenigstens eines Metalls der Gruppe VIII des Periodensystems der Elemente, abgeschieden auf dem Traeger umfasst und in dem wenigstens 80% der metallischen Katalysatorpartikel wenigstens eine Kontaktzone mit wenigstens einem anderen Partikel aufweist und die Partikel einen Umfang zwischen 50 und 500 Å haben, mit Hilfe eines Verfahrens, das wenigstens eine Imprägnierungsstufe mittels einer Lösung eines Vorläufers des Metalls auf den Träger umfasst, der auf eine Temperatur zwischen der Umgebungstemperatur und 200°C derart erwärmt ist, dass die Verdampfung der Lösung ab dem Kontakt mit dem Träger induziert wird.

9. Verfahren nach einem der Ansprüche 1 bis 8, bei dem die Imprägnierung des Katalysators Tropfen für Tropfen durchgeführt wird.

## Claims

1. Process for synthesis of hydrocarbons from a mixture that comprises carbon monoxide and hydrogen CO-H2, in the presence of a catalyst comprising a support, particles of at least one metal of group VIII of the periodic table that is (are) deposited on the support, **characterized in that** at least 80% of said metallic particles have at least one contact zone with at least one other particle and said particles have a size comprized between 50 Å and 500 Å.

2. Process according to one of claims 1, wherein at least 90% of the metal particles of the catalyst have at least one contact zone with at least one other particle.

3. Process according to one of claims 1 or 2, wherein the metal of group VIII of the catalyst is cobalt.

4. Process according to one of claims 1 to 3 wherein the catalyst in addition contains ruthenium.

5. Process according to one of claims 1 to 4, wherein the total pressure is between 0.1 and 15 MPa, the temperature is between 150 and 350°C, with an hourly volumetric flow rate of between 100 and 20,000 volumes of synthesis gas per volume of catalyst and per hour, with an H₂/CO ratio in the synthesis gas of between 1:2 and 5:1, preferably between 1.2:1 and 2.5:1.

6. Process according to one of claims 1 to 5 in which metallic particles have a size comprised between 60 Å and 400 Å.

7. Process according to one of claims 1 to 6 in which particles are in the form of essentially spherical agglomerates that are between 500 and 100.000 Å in size.

8. Preparation of a catalyst comprising a support, particles of at least one metal of group VIII of the periodic table that is (are) deposited on the support, wherein at least 80 percent of said metallic particles have at least one contact zone with at least one other particle and said particles have a size comprised between 50 ° Å and 500 ° Å, by means of a process comprising at least one step of impregnation with an aqueous solution of a precursor of said metal on said support heated to a temperature of between ambient temperature and 200°C, so as to induce the evaporation of said solution from the time of contact with the substrate.

9. Process according to one of claims 1 to 8 wherein the impregnation of said catalyst is performed drop by drop.
